# EUROPEAN PATENT APPLICATION

(11) **EP 1 484 036 A1**
(43) Date of publication of application: **08.12.2004**
(21) Application number: 03012798.9
(22) Date of filing: 05.06.2003
(51) Int. Cl.: A61F 2/38

(54) **Femoral component and artificial knee joint**

(71) Applicant: Nakashima Propeller Co., Ltd., Okayama-shi, Okayama (JP); Suguro, Toru, Chiba-shi, Chiba (JP)
(72) Inventor: Suguro, Toru, Chuo-ku, Chiba-shi, Chiba (JP)
(74) Representative: Bockhorni, Josef

(57) **Abstract**

A femoral component of an artificial knee joint that is to be attached to a distal end of a femur having a peg or pegs installed in the inner surface of the femoral component so as to be located in an area of a front half of the femoral component.

## Description

### BACKGROUND OF THE INVENTION

### 1. Field of the Invention

The present invention pertains to a femoral component and artificial knee joint that replace a knee joint.

### 2. Prior Art

Total knee arthroplasty (called "TKA") provides excellent clinical results as a method of treating severe knee joint degeneration associated with osteoarthritis, rheumatoid arthritis, etc. A typical artificial knee joints used for TKA is comprised of a femoral component and a tibial component. Of these, the femoral component, which is a metallic or ceramic implant (collectively called "metal implant"), is attached to the distal end of the femur. The femoral component must be anchored firmly to the distal end of the femur so that dislocation and loosening do not occur.

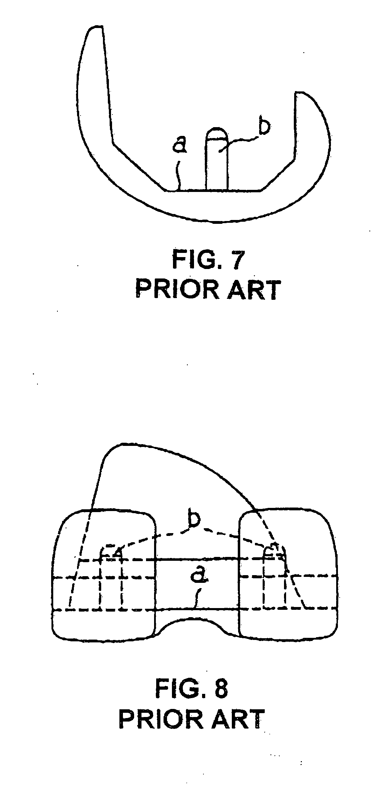

Figure 7 is a side view of a conventional femoral component, and Figure 8 is a rear view of the same.

In view of the above-described situations, the distal end of a femur is fitted to the femoral component, which has a polygonal shape when viewed from the side, after being orthopedically shaped so as to match the shape of the inner surface a of the femoral component. Two pegs b, one on the right side and one on the left, are provided in the inner surface a, and these pegs b are inserted into the bone for reinforcement of the anchor. The fitting between the femur and the metal implant is enhanced by way of applying bone cement between the two components, making the surface of the metal implant porous, or performing fine embossing on the surface of the metal implant

However, these methods for fitting reinforcement is strictly for increasing the fitting and adhesion of the two elements, and it does not increase the bone density that is necessary to prevent the loosening and supracondylar fractures that readily develop after TKA. According to recent reports, there is a reduction in bone density toward the front side of the condylar region of the femur following TKA. It has been said that the reasons for such a bone density reduction are a stress shielding based on a difference in the elastic modulus of bone and metal and a reduction in stress toward the front side following TKA.

On the other hand, it is reported that trabecula is produced due to the stress increase that is caused by stress redistribution after TKA, thus increasing bone density around the pegs and toward the back of the condylar region. Judging from this, the peg plays an important role in increasing bone density; and if this is the case, it can be said that providing a peg in the front region where the bone density tends to decease might inhibit the reduction of bone density of this area.

### SUMMARY OF THE INVENTION

The present invention is made based upon the above-described viewpoint, and it provides pegs in the front area of the condylar region.

More specifically, the present invention provides a femoral component of an artificial knee joint that is to be attached to a distal end of a femur and is provided with a peg installed in the inner surface of the femoral component; and in the present invention, the peg is provided in a front half area of the femoral component.

Bone density toward the front of the condylar region tends to decrease due to stress shielding following TKA. In particular, the reduction in bone density in the area toward the front is the reasons for the loosening of the femoral component and for supracondylar fractures. In the present invention, the pegs that increase the stress is provided in such an area; as a result, a reduction in bone density of this area is inhibited, and loosening and supracondylar fracture are prevented.

In the above structure, a sub-peg can be additionally provided behind the peg. With this structure having the sub-peg, an increase in bone density toward the back from the position of the sub-peg is expected. As a result, an increase in bone density over the entire area is expected. Moreover, the peg can be provided at laterally two (right and left) locations, and the sub-peg can be also provided at laterally two (right and left) locations. Such two sub-pegs are designed so as to be lower than the pegs in height, and they are spaced apart by a larger distance than the pegs. With this structure, both condylar regions can be supported with good balance in conformity to an actual biological knee joint

The present invention further provides an artificial knee joint that is comprised of the femoral component as described above and a tibial component. With a combination of a tibial component and a femoral component that prevents a reduction in bone density of the condylar region and also prevents loosening and supracondylar fracture, an excellent artificial knee joint that retains long-run performance following TKA can be obtained

### DETAILED DESCRIPTION OF THE DRAWINGS

Figure 1 is a side view of a femoral component, which is at the end of a femur, according to one embodiment of the present invention;
Figure 2 is a rear view thereof with the femur omitted;
Figure 3 is a top view thereof;
Figure 4 is a side view of a femoral component according to another embodiment of the present invention;
Figure 5 is a rear back view thereof;
Figure 6 is a top view thereof; [[and]]
Figure 7 is a side view of a femoral component of a conventional example; and
Figure 8 is a rear view of a femoral component of a conventional example.

### DETAILED DESCRIPTION OF THE INVENTION

Embodiments of the present invention will now be described below with reference to the accompanying drawings.

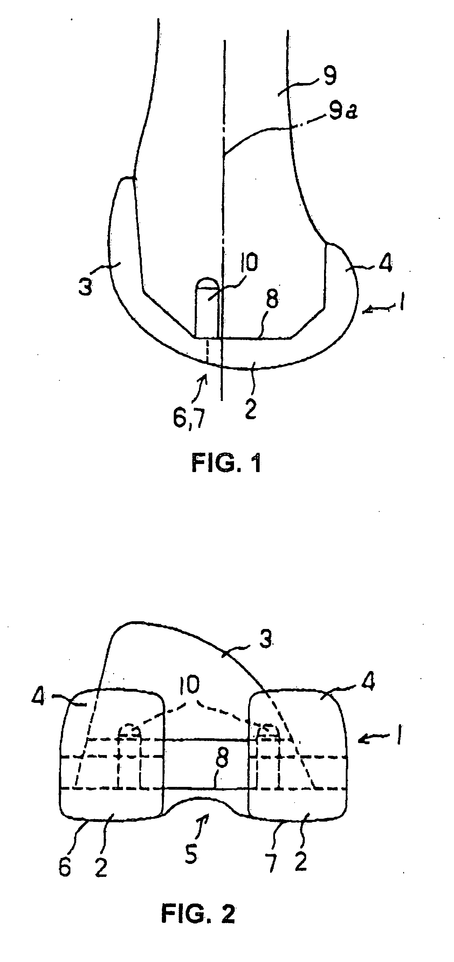

As seen from Figures 1 through 3, femoral component 1 of the shown embodiment is a metal or ceramic implant made from a biocompatible metal, such as titanium alloy or a biocompatible ceramic. When viewed from the side, as seen from Figure 1, the femoral component takes a substantially C-shape and has a tall front wall part 3 and a short rear wall part 4 with a curved part 2 in between as the bottom. The middle portion between the front of the curved part 2 and the rear wall 4 is omitted to make a condylar space 5 (see Figure 2). The outside surfaces on the left and right sides of the curved part 2 sandwiching the condylar space 5 are formed into a convex shape that makes a medial condylar region 6 and a lateral condylar region 7.

The inner surface 8 of the femoral component 1 is polygonal (a pentagon in this example) as seen from Figure 1, so that the distal end of femur 9 is orthopedically shaped and attached to this component. As previously described, fitting and adhesion of the femoral component 1 with the distal end of the femur 9 can be improved by way of filling bone cement at the surface of contact (between the femoral component 1 and the femur 9), making the surface of the inner surface 8 of the femoral component 1 porous, or applying embossing treatment. The medial condylar region 6 and lateral condylar region 7 are supported by an insert made of polyethylene, etc. of the tibial component that will be attached to the tibial side. These components can be the same as conventional ones, and their description is omitted here.

A peg 10, which will be inserted into the bone in order to provide a reliable attachment, is installed in the inner surface 8 of the femoral component 1. As seen from the shown embodiment, the peg 10 is provided in an area toward the front of the femoral component 1 compared to prior art femoral component. It is preferable that the position of the peg 10 be either close to or in front of the axis 9a of the femur 9. There are no special restrictions to the length or thickness of the peg 10.

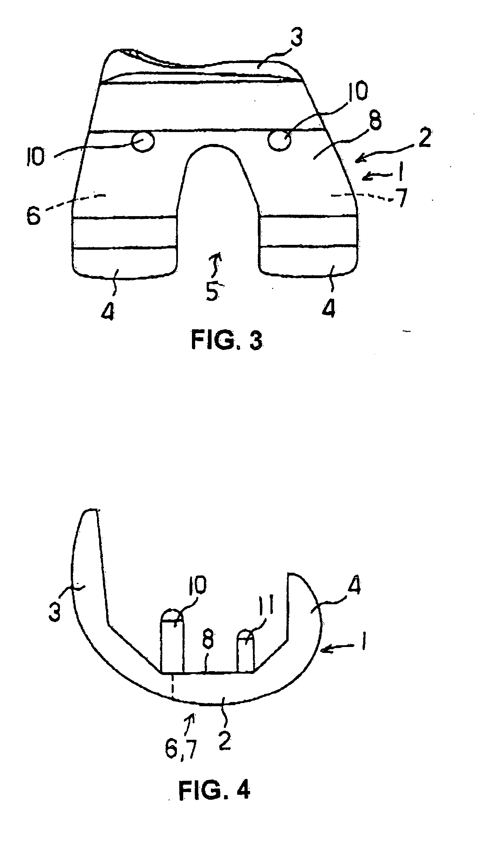

It is preferable that the peg 10 is provided so as to erect at right angles from a portion of the inner surface 8 that takes a substantially horizontal posture (which is the center of the pentagon) when a person with the femoral component 1 attached stands upright. With this positioning of the peg 10, the peg 10 is parallel to the axis 9a of the femur 9. The peg 10 is provided at two locations: on the right and left sides in the same position from the front and back as best seen from Figure 3. With the femoral component 1 with the structure described above, since the peg or pegs are provided in the front half area of the femoral component 1, the formation of trabecula in the area where there is a decrease in bone density after TKA is promoted, and the bone density in that area can increase.

Figures 4 through Figure 6 show another embodiment of the present invention.

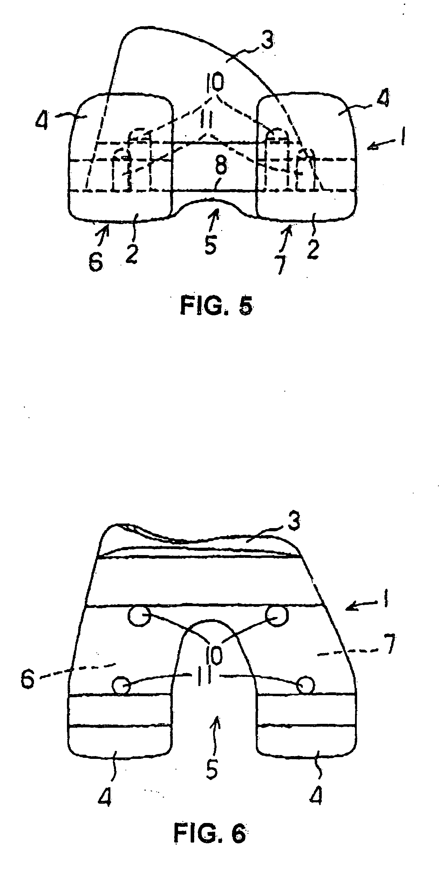

In the structure of this embodiment, sub-peg 11 is installed behind the peg(s) 10. The sub-peg 11 is installed in the area that is horizontal when a person wearing the femoral component 1 stands upright (that is in the center of the polygon). The sub-peg 11 is provided at two locations, on the right and left sides, and from the same distance from the front and the back as best seen from Figure 6.

It is preferable that each of two sub-pegs 11 be provided in the middle of the medial condylar region 6 and the lateral condylar region 7. Accordingly, in the shown embodiment, the distance between the right and left (two) sub-pegs 11 is wider than the distance between the right and left (two) pegs 10. In addition, the shown sub-peg(s) 11 is slightly lower than the peg(s) 10. The structure above is a mere example, and the present invention is not limited to this structure. In the structure of the femoral component 1 as described above, the sub-peg(s) 11 is installed behind the peg(s) 10; and thus, the formation of trabecula and an increase in bone density in the area toward the back of the femur can be expected.

The above-described femoral component 1 has condylar space 5 that is formed by omitting the center of the femoral component 1. However, though not illustrated, there is a femoral component that is called a "PS (Posterior Stabilized) type femoral component". In this femoral component, a space that holds a projection made in the tibial component is formed with a box shape so that the omitting portion has a PCL (Posterior Cruciate Ligament) function. It goes without saying that the present invention is applicable to such a PS type femoral component. The position of the peg(s) 10 in the PS type femoral component is, as in the shown embodiment, preferably close to or in front of bone axis 9a of the femur 9. When sub-peg(s) is provided, the position and shape thereof are the same as those in the shown embodiment.

With a combination of the femoral component as described above and a tibial component, an artificial knee joint is obtained. This knee joint, which is a combination of a tibial component and a femoral component that prevents a reduction in bone density of the condylar region and also prevents loosening and supracondylar fracture, retains long-run performance following TKA.

As seen from the above, in the femoral component of the present invention, the peg(s) is provided in an area thereof that corresponds to the front side of the condylar region of a femur. Accordingly, a reduction in bone density of this area toward the front, where bone density tends to decrease following TKA, is inhibited; and with sub-peg(s) provided behind the peg(s), an increase in bone density in the area behind where the sub-peg(s) is provided can be expected.

## Claims

1. A femoral component of an artificial knee joint that is to be attached to a distal end of a femur and is provided with a peg disposed in an inner surface of said femoral component, wherein said peg is provided in an area of a front half of said femoral component.

2. A femoral component according to claim 1, further comprising a sub-peg provided behind said peg.

3. A femoral component according to claim 2, wherein said peg is provided at laterally two locations and said sub-peg is provided at laterally two locations, said sub-pegs being lower than said pegs in height and being spaced by a larger distance than said pegs.

4. An artificial knee joint comprising a femoral component according to any one of claims 1 through 3 and a tibial component.

5. A femoral component of an artificial knee joint that is to be attached to a distal end of a femur and is provided with at least one peg disposed in an inner surface of said femoral component, wherein said at least one peg is provided in an area of said inner surface of said femoral component that corresponds to a front side of an axis of said femur to which said femoral component is attached.
